# EUROPEAN PATENT APPLICATION

(11) **EP 3 441 475 A1**
(43) Date of publication of application: **13.02.2019**
(21) Application number: 17185519.0
(22) Date of filing: 09.08.2017
(51) Int. Cl.: C12P 21/06, C12N 9/48, C12N 9/50, C12N 9/58

(54) **NEUTRAL HEAT-SENSITIVE SERINE PROTEASE DERIVED FROM O. CORVINA**

(71) Applicant: Barentzymes AS, 5528 Haugesund (NO)
(72) Inventor: SKOWRON, Piotr Mariusz, 80-303 Gdansk (PL); BRODZIK, Robert Wladyslaw, 80-126 Gdansk (PL); KOLLER, Klaus-Peter, 65812 Bad Soden (DE)
(74) Representative: Gruber, Daniel

(57) **Abstract**

The present invention relates to an isolated neutral heat-sensitive serine protease NHSSP of 25.6 to 31.2 kDa derived from the fungus Onygena corvina and a process for its preparation. The novel NHSSP is useful in many applications, particularly for use in detergent preparations, in molecular biology such as the preparation of nucleic acids like DNA and RNA, in the processing and manufacturing of food products and in the manufacturing of a pharmaceutical composition for wound debridement.

SEQUENCE LISTING

<110> Barentzymes AS

<120> Neutral Heat-Sensitive Serine Protease derived from O. corvina

<130> A2362DG

<160> 6

<170> BiSSAP 1.3.2

<210> 1
<211> 1209
<212> DNA
<213> Artificial Sequence

<223> "CDS 1...1209"

<220>
<223> CDS 1...1209

<400> 1
   atgggttgta tcaaggttat ctccgttttc ttggctgctg ttgctgctgt tgacgctaga 60
   gcttttttcc acaacaaggg tggtaacgac atcatcccaa actcctacat cgttgttatg 120
   aaggacggta tctccactca agagttcgag tcccacattt cttccgtttc tgctactcac 180
   gctaagagat ccactgagtt ggttggtcac aaggattcct tcaacatcaa cggttggaga 240
   gcttacaacg gtcacttcga tgctgctact ttggagtcca ttttgaacga cgacaacgtt 300
   aagtacgttg agcacgacag agttgttaag atctccgctt tgactactca gccaaacgct 360
   ccatcttggg gtttgggtag aatttctcac agatccccag gtaacaagga cttcgtttac 420
   gatgacactg ctggtcaggg tatcactatc tacggtgttg acactggaat cgacatcaac 480
   cacccagatt ttggtggtag agctagatgg ggaactaaca ctgttgacaa cgctaacaac 540
   gacggtcatg gtcatggtac tcatactgct ggtactttcg ctggtaacgc ttacggtatt 600
   gctaagaagg cttccgttgt tgctgttaag gttttgtctg cttctggttc cggttccaac 660
   gctggtgtta ttaagggtat cgactggtgt gttacagacg ctagatctaa gggtgctttg 720
   ggtaaggctg ctttgaactt gtctttgggt ggtggtttca accaggctac aaacgacgct 780
   gttactagag ctcagactgc tggaatcttc gttgctgttg ctgctggtaa cgataacaag 840
   gacgctagaa actactctcc agcttctgct ccagctgttt gtactgttgc ttcctccact 900
   atcgacgacc aaaagtcctc tttctctaac tggggttcca tcgttgacat ctacgctcca 960
   ggttccaaca ttatttccga tgctccaggt ggtggtgtta gaactatgtc tggtacttct 1020
   atggcttccc cacacgtttg tggtgctggt gctgctatgt tggctcaagg tgttccagtt 1080
   ggtcaggttt gtgacagatt gaagcagatc ggtaacgctg ttgttagaaa cccaggtact 1140
   tccactacaa acagattgtt gtataacggt tccggtcagg gttctcatca tcaccatcac 1200
   cactaatag                                                          1209

<210> 2
<211> 401
<212> PRT
<213> Onygena corvina

<400> 2
Met Gly Cys Ile Lys Val Ile Ser Val Phe Leu Ala Ala Val Ala Ala
1               5                   10                  15
Val Asp Ala Arg Ala Phe Phe His Asn Lys Gly Gly Asn Asp Ile Ile
            20                  25                  30
Pro Asn Ser Tyr Ile Val Val Met Lys Asp Gly Ile Ser Thr Gln Glu
        35                  40                  45
Phe Glu Ser His Ile Ser Ser Val Ser Ala Thr His Ala Lys Arg Ser
    50                  55                  60
Thr Glu Leu Val Gly His Lys Asp Ser Phe Asn Ile Asn Gly Trp Arg
65                  70                  75                  80
Ala Tyr Asn Gly His Phe Asp Ala Ala Thr Leu Glu Ser Ile Leu Asn
                85                  90                  95
Asp Asp Asn Val Lys Tyr Val Glu His Asp Arg Val Val Lys Ile Ser
            100                 105                 110
Ala Leu Thr Thr Gln Pro Asn Ala Pro Ser Trp Gly Leu Gly Arg Ile
        115                 120                 125
Ser His Arg Ser Pro Gly Asn Lys Asp Phe Val Tyr Asp Asp Thr Ala
    130                 135                 140
Gly Gln Gly Ile Thr Ile Tyr Gly Val Asp Thr Gly Ile Asp Ile Asn
145                 150                 155                 160
His Pro Asp Phe Gly Gly Arg Ala Arg Trp Gly Thr Asn Thr Val Asp
                165                 170                 175
Asn Ala Asn Asn Asp Gly His Gly His Gly Thr His Thr Ala Gly Thr
            180                 185                 190
Phe Ala Gly Asn Ala Tyr Gly Ile Ala Lys Lys Ala Ser Val Val Ala
        195                 200                 205
Val Lys Val Leu Ser Ala Ser Gly Ser Gly Ser Asn Ala Gly Val Ile
    210                 215                 220
Lys Gly Ile Asp Trp Cys Val Thr Asp Ala Arg Ser Lys Gly Ala Leu
225                 230                 235                 240
Gly Lys Ala Ala Leu Asn Leu Ser Leu Gly Gly Gly Phe Asn Gln Ala
                245                 250                 255
Thr Asn Asp Ala Val Thr Arg Ala Gln Thr Ala Gly Ile Phe Val Ala
            260                 265                 270
Val Ala Ala Gly Asn Asp Asn Lys Asp Ala Arg Asn Tyr Ser Pro Ala
        275                 280                 285
Ser Ala Pro Ala Val Cys Thr Val Ala Ser Ser Thr Ile Asp Asp Gln
    290                 295                 300
Lys Ser Ser Phe Ser Asn Trp Gly Ser Ile Val Asp Ile Tyr Ala Pro
305                 310                 315                 320
Gly Ser Asn Ile Ile Ser Asp Ala Pro Gly Gly Gly Val Arg Thr Met
                325                 330                 335
Ser Gly Thr Ser Met Ala Ser Pro His Val Cys Gly Ala Gly Ala Ala
            340                 345                 350
Met Leu Ala Gln Gly Val Pro Val Gly Gln Val Cys Asp Arg Leu Lys
        355                 360                 365
Gln Ile Gly Asn Ala Val Val Arg Asn Pro Gly Thr Ser Thr Thr Asn
    370                 375                 380
Arg Leu Leu Tyr Asn Gly Ser Gly Gln Gly Ser His His His His His
385                 390                 395                 400
His

<210> 3
<211> 837
<212> DNA
<213> Artificial Sequence

<223> "CDS 1...837"

<220>
<223> CDS 1...837

<400> 3
gctttgacta ctcagccaaa cgctccatct tggggtttgg gtagaatttc tcacagatcc 60

ccaggtaaca aggacttcgt ttacgatgac actgctggtc agggtatcac tatctacggt 120

gttgacactg gaatcgacat caaccaccca gattttggtg gtagagctag atggggaact 180

aacactgttg acaacgctaa caacgacggt catggtcatg gtactcatac tgctggtact 240

ttcgctggta acgcttacgg tattgctaag aaggcttccg ttgttgctgt taaggttttg 300

tctgcttctg gttccggttc caacgctggt gttattaagg gtatcgactg gtgtgttaca 360

gacgctagat ctaagggtgc tttgggtaag gctgctttga acttgtcttt gggtggtggt 420

ttcaaccagg ctacaaacga cgctgttact agagctcaga ctgctggaat cttcgttgct 480

gttgctgctg gtaacgataa caaggacgct agaaactact ctccagcttc tgctccagct 540

gtttgtactg ttgcttcctc cactatcgac gaccaaaagt cctctttctc taactggggt 600

tccatcgttg acatctacgc tccaggttcc aacattattt ccgatgctcc aggtggtggt 660

gttagaacta tgtctggtac ttctatggct tccccacacg tttgtggtgc tggtgctgct 720

atgttggctc aaggtgttcc agttggtcag gtttgtgaca gattgaagca gatcggtaac 780

gctgttgtta gaaacccagg tacttccact acaaacagat tgttgtataa cggttcc 837


<210> 4
<211> 279
<212> PRT
<213> Onygena corvina


<400> 4
Ala Leu Thr Thr Gln Pro Asn Ala Pro Ser Trp Gly Leu Gly Arg Ile
1               5                   10                  15
Ser His Arg Ser Pro Gly Asn Lys Asp Phe Val Tyr Asp Asp Thr Ala
            20                  25                  30
Gly Gln Gly Ile Thr Ile Tyr Gly Val Asp Thr Gly Ile Asp Ile Asn
        35                  40                  45
His Pro Asp Phe Gly Gly Arg Ala Arg Trp Gly Thr Asn Thr Val Asp
    50                  55                  60
Asn Ala Asn Asn Asp Gly His Gly His Gly Thr His Thr Ala Gly Thr
65                  70                  75                  80
Phe Ala Gly Asn Ala Tyr Gly Ile Ala Lys Lys Ala Ser Val Val Ala
                85                  90                  95
Val Lys Val Leu Ser Ala Ser Gly Ser Gly Ser Asn Ala Gly Val Ile
            100                 105                 110
Lys Gly Ile Asp Trp Cys Val Thr Asp Ala Arg Ser Lys Gly Ala Leu
        115                 120                 125

Gly Lys Ala Ala Leu Asn Leu Ser Leu Gly Gly Gly Phe Asn Gln Ala
    130                 135                 140
Thr Asn Asp Ala Val Thr Arg Ala Gln Thr Ala Gly Ile Phe Val Ala
145                 150                 155                 160
Val Ala Ala Gly Asn Asp Asn Lys Asp Ala Arg Asn Tyr Ser Pro Ala
                165                 170                 175
Ser Ala Pro Ala Val Cys Thr Val Ala Ser Ser Thr Ile Asp Asp Gln
            180                 185                 190
Lys Ser Ser Phe Ser Asn Trp Gly Ser Ile Val Asp Ile Tyr Ala Pro
        195                 200                 205
Gly Ser Asn Ile Ile Ser Asp Ala Pro Gly Gly Gly Val Arg Thr Met
    210                 215                 220
Ser Gly Thr Ser Met Ala Ser Pro His Val Cys Gly Ala Gly Ala Ala
225                 230                 235                 240
Met Leu Ala Gln Gly Val Pro Val Gly Gln Val Cys Asp Arg Leu Lys
                245                 250                 255
Gln Ile Gly Asn Ala Val Val Arg Asn Pro Gly Thr Ser Thr Thr Asn
            260                 265                 270
Arg Leu Leu Tyr Asn Gly Ser
        275

<210> 5
<211> 21
<212> DNA
<213> Artificial Sequence

<223> "Primer P1"

<220>
<223> Primer P1

<400> 5
gactggttcc aattgacaag c                                              21

<210> 6
<211> 19
<212> DNA
<213> Artificial Sequence

<223> "Primer P2"

<220>
<223> Primer P2

<400> 6
gcgtgaatgt aagcgtgac                                                 19

## Description

### FIELD OF THE INVENTION

The present invention relates to an isolated neutral heat-sensitive serine protease (NHSSP) of 25.6 to 31.2 kDa derived from the fungus Onygena corvina and a process for its preparation. The novel NHSSP is useful in many applications, particularly for use in detergent preparations, in molecular biology such as the preparation of isolated nucleic acids like DNA and RNA, in the processing and manufacturing of food products, and in the manufacturing of a pharmaceutical composition for wound debridement.

### BACKGROUND

Proteases belong to a very important class of hydrolytic enzymes, which find applications in many different industrial sectors and are suitable, e.g., as additives in detergent compositions, as reagents in molecular biology, and for the processing and manufacturing of food products, pharmaceutical products or diagnostic products, and, finally also in waste management.

Serine proteases of the subtilisin family or subtilisins produced by Bacillus species form one of the largest group of industrial proteases. These enzymes are commercially important as protein degrading components. A lot of efforts has been undertaken to identify enzymes of natural sources or to generate variants of natural occurring enzymes by mutagenesis to exhibit features, which may better meet the needs of specific industrial applications. Such features include an improved catalytic efficiency or stability with respect to temperature, pH value or the presence of other ingredients, such as, detergents, chaotropic or oxidizing agents, which may generally affect or inhibit enzymatic activity of proteases.

Despite of molecular biological approaches, naturally occurring microorganisms, yeasts and fungi still build an enormous reservoir for the identification of proteases which exhibit feature combinations, which are suitable for specific industrial applications. E.g., many alkaline serine proteases (EC 3.4.21) and genes encoding such enzymes have been isolated from eukaryotic organisms, including yeast and fungi. In addition, international patent application WO2015158719A1 discloses several endopeptidases of the MEROPS family S8 from Onygena corvina, which have been subject to investigation for their suitability in the degradation of keratinaceous materials.

Due to ecological and economical needs, which may also be manifested by governmental regulations, there is always the need to reduce waste production and to save energy.

Since proteases are important ingredients in detergent compositions, it would be desirable to reduce energy input by effectively performing the process at relatively low temperatures. In addition, it may be desirable to avoid high temperatures in such a process in order to deactivate the employed protease, if protease activity or protein needs to be removed for whatever purpose in the performance of further process steps. Furthermore, serine proteases for use in laundry and dish detergent compositions should also be stable in the presence of detergents, for which in general terms proteases of the subtilisin family are known to be relatively robust.

There is still a need for the provision of alternative proteases, which effectively degrade proteinaceous material, whereby the active protease is active at moderate temperatures and at a neutral pH value. For some applications, it would be furthermore advantageous to provide such a protease, which could be deactivated at relatively moderate temperatures.

Therefore, it is an object of instant invention to provide an isolated serine protease, which is capable of removing proteinaceous material at moderate temperatures, optionally in the presence of detergents, and at a neutral pH, whereby the protease can be deactivated by means of a relatively moderate temperature increase and can be produced in high-yielding hosts and its down-stream processing and isolation, e.g. separation of fermentation broth is easy to perform.

New proteases and compositions comprising such enzymes should be able to effectively work in the required pH and temperature range thereby remaining stable under appropriate conditions, including chemical and, optionally, mechanical conditions of the process wherein they are applied. Furthermore, it is required that a new serine protease can be produced in an appropriate high amount using a cost-effective down-stream process which allows easy purification from the fermentation broth.

### SUMMARY OF THE INVENTION

Surprisingly, it was found that a protease which is generally described in WO2015158719A1 and which was defined to belong to the alkaline serine proteases of the subtilisin type, especially the S8 family (cf. GenBank KP290860.1, UniProtKB A0A0B4VM82) unexpectedly exhibits enzymatic activity at neutral pH values. The truncated mature enzyme is active at neutral to acidic pH values of 5.0 to 7.5 and at a temperature of 20 to 45°C. Furthermore, it is unexpected that said protease can be fully deactivated by a relatively moderate increase in temperature (of about 15°C from the temperature exhibiting the optimum performance), which allows to deactivate said protease at a temperature of 50°C, preferably 55°C, more preferred 60°C, or more within a very short time period, i.e., within minutes. These unexpected features predestine the suitability of this novel matured enzyme for specific applications as subsequently described, e.g., applications, where deactivation of proteolytic activity shall be performed at moderate temperature.

Therefore, a first object of the present invention is a process for the preparation of an isolated polypeptide having a calculated molecular weight of 25.6 to 31.2 kDa, preferably of 27 to 29.8 kDa and neutral heat-sensitive serine protease (NHSSP) activity, said process comprising the steps of
(a) culturing a host cell comprising a nucleic acid molecule, which comprises a polynucleotide sequence encoding a neutral serine protease selected from the group consisting of:
   (i) a nucleic acid sequence encoding a polypeptide comprising the amino acid sequence of SEQ ID No. 2 or 4;
   (ii) a nucleic acid sequence encoding a polypeptide having at least 87%, preferably at least 90%, more preferred at least 95% identity to the amino acid sequence of SEQ ID No. 2 or 4;
   (iii) a nucleic acid sequence comprising the coding sequence of the nucleotide sequence of SEQ ID No. 1 or 3;
   (iv) a nucleic acid sequence comprising a coding sequence which differs from the coding sequence of the nucleic acid sequence of (iii) due to the degeneracy of the genetic code; and
   (v) a nucleic acid sequence encoding a polypeptide having serine protease activity as deposed under DSM No. 32492
   (vi) a nucleic acid sequence which hybridizes under stringent conditions to the nucleic acid sequence as defined in (iii) to (v); and
(b) recovering said polypeptide having serine protease activity from the culturing broth;
(c) maturing said polypeptide or said recovered polypeptide; and
(d) isolating said matured polypeptide having NHSSP activity, and wherein, optionally, step (c) may take place before step (b).

In the preferred various embodiments of the invention the polypeptide having NHSSP activity is recombinant.

In a preferred embodiment of the process of the invention an expression vector comprises the polynucleotide sequence encoding a neutral serine protease enzyme, which is operably linked to one or more regulatory sequences capable of directing the expression of said polypeptide in said host cell. The host cell which is suitable in the process of the invention is pro- or eukaryotic, preferably eukaryotic, and more preferred derived from fungus or yeast.

The culture medium of the production host can be used as such, or the host cells may be removed, and/or it may be concentrated, filtrated or fractionated. It may also be dried. Preferably, particles such as cellular debris is removed by centrifugation or filtration or other means known to the skilled person, if such particles interfere with subsequent purification steps.

In another embodiment of the method of the invention, the said polynucleotide sequence further encodes one or more signal peptides and/or tag peptides which may facilitate purification of the polypeptide in the subsequent recovery step (b) or isolation step (d).

It is a second object of instant invention to provide an isolated neutral heat-sensitive serine protease having a calculated molecular mass of 25.6 to 31.2 kDa, preferably of 27 to 29.8 kDa, which is obtainable by the process of the invention and which exhibits at least one of the following features:
- a pH optimum of its enzymatic activity at pH 5.0 to 7.5 and 40°C using 30 min reaction time and azocasein as a substrate in 20mM potassium phosphate buffer, preferably under the assay conditions set forth in example 4.C.1;
- a temperature optimum of its enzymatic activity at 35 to 45°C and pH 5.5 using 30 min reaction time and azocasein as a substrate in 50mM potassium phosphate buffer, preferably under the assay conditions set forth in example 4.C.2.

The term "serine protease" means according to the invention an enzyme classified as EC 3.4.21 by the Nomenclature of the International Union of Biochemistry and Molecular Biology.

The term "neutral serine protease activity" means a hydrolytic activity on a polypeptidic substrate such as azocasein, casein, haemoglobin, keratin, or BSA, at a pH value of about 5.0 to 7.5, preferably 5.5 to 7.0. Methods for analyzing proteolytic activity of serine proteases are well-known in the art and are referred to, e.g., by Gupta et al. (2002). The term "neutral serine protease" according to instant invention preferably means that the pH optimum of the active enzyme is in the range of pH 5.5 to 7.5.

The term "heat-sensitive" according to instant invention preferably means that the half-life of the active enzyme is reduced by a factor 100 or more at a temperature, which exceeds at least 10 to 15 °C the temperature optimum of said enzyme, thereby leading to complete inactivation of the enzyme within a time period of less than 100 minutes, preferably less than 10 minutes.

One preferred embodiment of the second object of the invention is the isolated mature NHSSP of SEQ ID No. 4 or a polypeptide having at least 87%, more preferred at least 90%, even more preferred at least 95% sequence identity with SEQ ID No. 4.

It is another object of instant invention to provide an enzyme preparation or a detergent preparation comprising the isolated NHSSP ("neutral heat-sensitive serine protease") of the invention. In another embodiment, the enzyme preparation of the invention further comprises an effective amount of one or more suitable additives selected from the group of stabilizers, buffers, cofactors, ions, surfactants, chelators, and preservatives. In another embodiment, the detergent preparation of the invention further comprises an effective amount of one or more suitable additives selected from the group of stabilizers, buffers, cofactors, ions, enzymes, chelators, and preservatives. In yet another embodiment of the invention the enzyme or detergent composition is in the form of liquid, powder or granulate, optionally in a compressed form.

It is yet another object of instant invention to use the isolated polypeptide having NHSSP activity of the invention, the enzyme preparation of the invention, or the detergent preparation of the invention in a proteolytic process at a pH value of between pH 5.0 or 5.5 and pH 7.5 or 7.0, preferably at a temperature of 20 to 50°C.

It is still another object of instant invention to use the isolated polypeptide having NHSSP activity of the invention or the enzyme preparation of the invention for the preparation of a detergent composition, preferably a detergent composition, which shall be used at a pH of 5.0 to 7.5, preferably at a temperature of 20 to 50°C.

It is still another object of instant invention to use the isolated polypeptide having NHSSP activity of the invention or the enzyme preparation of the invention for the preparation or processing of food or food products.

It is yet another object of instant invention to use the isolated polypeptide having NHSSP activity of the invention, the enzyme preparation of the invention, or the detergent preparation of the invention in the purification of nucleic acid molecules.

A method of performing a proteolytic process at a pH value of pH 5.0 to 7.5 and at a temperature of 20 to 50°C, wherein a polypeptide of a calculated molecular mass of 25.6 to 31.2 kDa having NHSSP activity is employed, optionally in the presence of one or more detergents, comprising the polypeptide selected from the group consisting of
(i) the amino acid sequence as defined in SEQ ID No. 4; and
(ii) the amino acid sequence having at least 87% identity to the amino acid sequence of SEQ ID No. 4;
said method optionally further comprising the step of terminating said proteolytic process by deactivating said protease at a temperature of 55°C or more, preferably, wherein said protease is employed in the presence of a Ca²⁺ or Mg²⁺ complexing agent.

The term "detergent" according to instant invention preferably means any detergent known to the skilled person, which is suitable in the performance of proteolytic processes, e.g., in household and molecular biology.

The term "Ca²⁺ or Mg²⁺ complexing agent" according to instant invention preferably means any agent, which is known to the skilled person to complex or chelate calcium or magnesium ions and which is suitable in the proteolytic process of the invention, which optionally includes the presence of a detergent, e.g., EDTA (ethylenediamine tetraacetate), NTA (triacetate), triphosphate, polycarboxylate, phosphonate, gluconate, citrate or zeolith.

### BRIEF DESCRIPTION OF FIGURES

### SEQUENCE LISTING

SEQ ID No. 1: Synthetic polynucleotide sequence encoding the polypeptide of Seq ID No. 2 including optimized codon usage for gene expression in Pichia
SEQ ID No. 2: Polypeptide sequence of a neutral heat-sensitive serine protease derived from O. corvina
SEQ ID No. 3: Synthetic polynucleotide sequence encoding the polypeptide of Seq ID No.4 including optimized codon usage for gene expression in Pichia
SEQ ID No. 4: Polypeptide sequence of the mature neutral heat-sensitive serine protease derived from O. corvina
SEQ ID No. 5: Oligonucleotide Primer P1
SEQ ID No. 6: Oligonucleotide Primer P2

### DEPOSITION OF BIOLOGICAL MATERIAL

P. pastoris strain NHSSP Pichia Pink Strain 1 clone 101 including plasmid pPink_HC/NHSSP was deposited by Arctic Earth Science AS at the Leibniz-Institut Deutsche Sammlung von Mikroorganismen und Zellkulturen GmbH (DSMZ), Inhoffenstrasse 7 B, 38124 Braunschweig, Germany on April 25, 2017 under accession number DSM 32492.

### DETAILED DESCRIPTION

The present invention provides an isolated neutral heat-sensitive serine protease of fungal origin, which shows broad substrate specificity, is stable in a wide pH range and has a temperature optimum of 35 to 45°C, which is heat-sensitive at temperatures above 50°C. The enzyme is ideally suitable for detergent applications, withstanding oxidizing and chelating agents and being effective at low enzyme levels in detergent solutions. Particularly, the serine protease is active at temperatures as from 20°C, the preferred range being from 30°C to 45°C. Thus, the present invention provides an alternative neutral serine protease for use in detergent compositions, the manufacturing of detergent wipes, polynucleotide purification, the processing of food and feed, wound debridement, the manufacturing of pharmaceutical compositions for wound debridement, the manufacturing of detergent compositions for biofilm (i.e., proteinaceous films) removal, especially for medical devices, and many other applications. The neutral serine protease can be effectively produced in high-yielding hosts and its down-stream processing, e.g. separation of fermentation broth is easy to perform.

Serine proteases can be classified using group specific inhibitors of synthetic or natural origin. One group of natural inhibitors are serpins (abbreviated from serine protease inhibitors), such as antithrombin and alpha 1-antitrypsin. Artificial synthetic inhibitors include 3,4-dichloroisocoumarin (3,4-DCI), diisopropylfluoro-phosphate (DFP), phenylmethylsulfonyl fluoride (PMSF) and tosyl-L-lysine chloromethyl ketone (TLCK). Some of the serine proteases are inhibited by thiol reagents such as p-chloromercuribenzoate (PCMB) due to the presence of a cysteine residue near the active site. Thus, the serine protease activity can be determined in an assay based on cleavage of a specific substrate or in an assay using any protein containing substrate with or without a specific inhibitor of serine proteases under suitable conditions.

Most serine proteases are generally active at neutral or alkaline pH, with an optimum between pH 7 and 11, and have broad substrate specificity. "Alkaline serine protease" generally means enzymes that are active and stable at pH 9 to pH 11 or even at pH 10 to 12.5 (Shimogaki et al., 1991) and have an isoelectric point at about pH 9. Those represent the largest subgroup of commercially used serine proteases. The molecular masses of alkaline serine proteases range between 15 and 35 kDa. The temperature optima of the natural serine proteases are around 60°C (Rao et al.,1998).

Yeast, fungi or microbial strains capable of producing protease activity can be screened and the activity on different substrates can be determined. Chosen strains can be cultivated on a suitable medium. After a sufficient amount of an interesting serine protease has been produced, the enzyme can be isolated or purified and its properties can be more thoroughly characterized. Alternatively, genes encoding serine proteases in various organisms have been isolated and the amino acid sequence encoded by the genes have been compared with the amino acid sequences of the serine protease isolated and characterized.

Unexpectedly, it was found that the isolated serine protease derived from O. corvina as hereinafter described exhibits an activity optimum at about pH 6,8 and at a temperature of about 40°C. Even more surprising, it was found that the enzyme is heat-sensitive above a temperature of about 50°C. Therefore, the enzyme was named "neutral heat-sensitive serine protease" ("NHSSP").

The NHSSP derived from O. corvina can essentially be purified by using conventional methods of enzyme chemistry, such as salt preparation, ultrafiltration, ion exchange chromatography, affinity chromatography, gel filtration, and hydrophobic interaction chromatography. Purification can be monitored by methods such as protein determination, enzyme activity assays, and by SDS polyacrylamide gel electrophoresis. The enzyme activity and stability of the purified enzyme at various temperature and pH values as well as the molecular mass and the isoelectric point and other biophysical-chemical properties can be determined.

One particular way how to purify the neutral heat-sensitive serine protease derived from O. corvina is demonstrated in Example 3.

There are many ways to determine the molecular mass of a protein. The molecular mass of purified serine proteases can be determined by mass spectrometry or on SDS-PAGE according to Laemmli (1970). The molecular mass can also be predicted by calculation from the amino acid sequence of the enzyme "calculated molecular mass". Generally, mature serine proteases have a molecular mass between 20 to 35 kDa, typically around 25 to 30 kDa (Rao et al.,1998). The NHSSP according to instant invention has a calculated molecular mass of 28.361 kDa and an apparent molecular weight of 34 kDa as determined by SDS-PAGE (cf. Example 4A).

Generally, serine proteases are synthesized as inactive "zymogenic precursors" or "zymogens" in the form of a pre-proenzyme, which are activated by removal of the signal sequence (secretion signal peptide or pre-peptide) and the pro-sequence (pro-peptide) to yield an active mature enzyme (Chen and Inouye, 2008). This activation process implies the activity of a protease and may result by autocatalytic processing of the serine protease zymogen or the presence of other proteolytic activity. The pro-sequence may be cleaved for example during post-translational phases of the production, in the spent culture medium or during storage of the culture medium or enzyme preparation. Activation of the proenzyme may also be facilitated by adding a proteolytic enzyme (e.g., trypsin) capable of converting the inactive proenzyme into an active mature enzyme into the culture medium where the host organism is cultivated or adding the proteolytic enzyme to the culture supernatant after the cultivation process. The shortening of the enzyme may also be achieved by truncating the gene encoding the polypeptide prior to transforming it to the production host. The prosequence has a calculated molecular mass of 39.937 kDa and comprises 382 amino acids.
The term "mature" according to instant invention means the enzyme which after removal of the signal sequence and pro-peptide comprises the essential amino acids which enable enzymatic or catalytic activity. With respect to the NHSSP derived from O. corvina of instant invention it preferably means the amino acid sequence according to SEQ ID No. 4. The mature NHSSP has a calculated molecular mass of 28.361 kDa and comprises 279 amino acids.

The determination of the temperature optimum of a serine protease can generally be determined in a suitable proteolytic assay using a suitable buffer at different temperatures and constant pH value, e.g., by using azocasein as a substrate as described in Example 4.C.2. The determination of the pH optimum of a serine protease can generally be determined in a suitable proteolytic assay using a suitable buffer system at different pH values and constant temperature, e.g., by using azocasein as a substrate as described in Example 4.C.1. Alternatively, other suitable substrates and buffer systems may be used as described in the literature (cf., e.g., Gupta et al., 2002).

Protease activity measurement is generally based on the degradation of soluble substrates. In some detergent application proteases have to work on substrates which are at least partly insoluble. Thus, an important parameter for a detergent protease is the ability to bind to and hydrolyse these insoluble fragments.

Another parameter for the selection of detergent proteases is its isoelectric point or pl value. The detergent proteases perform best when the pH value of the detergent solution in which it works is approximately the same as the pl value for the enzyme. pl can be determined by isoelectric focusing on an immobilized pH gradient gel composed of polyacrylamide, starch or agarose or by estimating the pl from the amino acid sequence, for example by using the pl/MW tool at ExPASy server (http://expasy.org/tools/pi tool.html; Gasteiger et al., 2003) or, as done here by using the information given by the Mascot Search Results provided by Matrix Sciences Inc., Boston along with the MS/MS analysis of peptide fragment pattern. The pl for the mature NHSSP was calculated to be 8.7.

The N-terminus of the purified protease as well as internal peptides can be sequenced according to Edman degradation chemistry (Edman and Begg, 1967) as described in Example 4B or by other methods described in the literature.

Generally, fungal alkaline serine proteases are advantageous to the bacterial proteases due to the ease of down-stream processing to produce a microbe-free enzyme or enzyme composition. Optionally, mycelium can be removed by filtration, centrifugation or other methods known to the skilled person prior to the purification of the enzyme.

The present invention relates to a neutral heat-sensitive serine protease, which has a good performance in the presence of detergents with varying properties, at a temperature range from 10°C to 50°C.

Within the meaning of the present invention 'good performance in presence of detergent' means that the NHSSP of the invention, works at lower temperature or pH ranges and/or higher specific activity than other commercially available serine proteases. In other words, good performance means that the enzyme is capable of degrading or removing proteinaceous material at low to moderate temperatures and/or pH values, but especially at lower temperature or pH value ranges than the present available commercial products, including Proteinase K (obtainable from Sigma Aldrich or ThermoFisher).

From the experimental results it can be concluded that the neutral serine protease of the invention is capable of satisfying the greatly varying demands of detergent customers and detergent industry and industry providing laundry detergents and is well suited to the requirements of future regulations and customer habits.

The term "identity" according to instant invention means with respect to a polypeptide sequence the grade of identity in % between two amino acid sequences compared to each other within the corresponding sequence region having approximately the same amount of amino acids. For example, the identity of a full-length or a mature sequence of the two amino acid sequences may be compared. The amino acid sequences of the two molecules to be compared may differ in one or more positions, which however does not alter the biological function or structure of the molecules. Such variation may occur naturally because of different host organisms or mutations in the amino acid sequence or they may be achieved by specific mutagenesis. Other variations may result from deletion, substitution, insertion, addition or combination of one or more positions in the amino acid sequence. The identity of the sequences is measured and expressed by using ClustalW alignment (e.g., www.ebi.ac.uk/Tools/Clustalw). The matrix parameters used are as follows: BLOSUM, Gap pen:10, Gap extension: 0.5.

Preferably, the NHSSP according to instant invention is obtainable from Onygena sp., preferably from Onygena corvina or may be obtained by expression of a synthetic gene in a heterologous host of the known DNA sequence.

One preferred embodiment of the invention is the polypeptide having NHSSP activity and an amino acid sequence of the mature enzyme as defined in SEQ ID No 4. The mature enzyme lacks the signal sequence or prepeptide and the prosequence or propeptide. However, within the scope of the invention is also the use of the full-length enzyme having SEQ ID No. 2 including the signal sequence (prepeptide) and propeptide and the mature enzyme as well as the proenzyme form lacking the signal sequence (prepeptide). In addition, fragment analysis followed by MS/MS analysis revealed that NHSSP also lacks amino acids at its C-terminus.

The present invention relates to a neutral serine protease enzyme, the mature form of which has a calculated molecular mass or molecular weight between 25.6 and 31.2 kDa, preferably between 27 and 29.8 kDa. The most preferred MW is the calculated molecular mass of the NHSSP being 28.4 kDa for the mature polypeptide obtained by LC-MS/MS analysis of the purified protein (Mascot Software http://www.matrixscience.com Mascot Search Results, Matrix Sciences Inc., Boston, USA

The enzyme of the invention is effective in degrading proteinaceous material at a broad temperature range. The optimal temperature of the enzyme is from 15°C to 50°C (about 20% of the maximum activity), preferably from 35°C to 45°C (at least about 40% of the maximum activity), and most preferably at 38°C to 42°C when measured at pH 6.5 using 30 min reaction time and azocasein as a substrate as described in Example 4.C.2.

According to one preferred embodiment of the invention the NHSSP has the pH optimum at a pH range from at least pH 5.0 to pH 7.5, showing over 40% of the maximum activity at pH 6.8 at 40°C using 30 min reaction time and azocasein as a substrate as described in Example 4.C.1. In particular, the pH optimum is between pH 5.4 and pH 7.3 (about 60% of the maximum activity), and more preferably between pH 6, and pH 7 (about 80% of the maximum activity), and most preferably at pH 6.8 at 40°C.

The NHSSP of the invention has a "good performance in the presence of detergent", i.e. is capable of degrading or removing proteinaceous films, stains or material in the presence of detergent at low temperature ranges, particularly under conditions of neutral pH, preferably at a pH range from at least pH 5.0 to pH 7.5. In the presence of a detergent the NHSSP of the invention functions between 10°C and 50°C, preferably at or below 40°C.

Oligonucleotides synthesized on the amino acid sequence of N-terminal or tryptic peptides of the purified enzyme or a PCR product obtained by using the above oligonucleotides can be used as probes in isolation of cDNA or a genomic gene encoding the serine protease of the invention. The probe may be designed also based on the known nucleotide or amino acid sequences of homologous serine proteases. The serine protease clones may also be screened based on activity on plates containing a specific substrate for the enzyme or by using antibodies specific for a serine protease.

According to a preferred embodiment of the invention the NHSSP is encoded by an isolated polynucleotide sequence which hybridizes under stringent conditions with a polynucleotide or probe sequence included in plasmid pPink_HC/NHSSP comprising the nucleotide sequence SEQ ID No. 1 in P. pastoris NHSSP Pichia Pink Strain 1 clone 101, deposited at the Leibniz-Institut Deutsche Sammlung von Mikroorganismen und Zellkulturen (DSMZ) under accession number DSM 32492.

Based on the amino acid sequence of proteases derived from Genbank entry No. KP29860 a recombinant protein with C-terminal His6 tag was designed exhibiting an optimized codon usage for P. pastoris and the de novo synthesis of corresponding cDNAs was obtained from GeneArt (Thermo Fisher Scientific). The obtained gene strand was cloned into vector pPink-HC (Pichia Pink system, Thermo Fisher Scientific) into the sites EcoRI (5'-end) and Kpnl (3'-end). Based on in silico simulation native signal peptides (SP) were used to drive secretion to the culture medium.

Standard molecular biology methods can be used in the isolation of cDNA or a genomic DNA of the host organism, e.g. the methods described in the molecular biology handbooks, such as Sambrook and Russell (2001).

Hybridization with a DNA probe consisting of more than 200 nucleotides, is usually performed at "high stringency" conditions, i.e. hybridization at a temperature, which is 20 to 25°C below the calculated melting temperature (Tm) of a perfect hybrid, the Tm calculated according to Bolton and McCarthy (1962). Pre-hybridization and hybridization are performed at least at 65°C in 6xSSC, 5xDenhardt's reagent, 0.5% SDS, 100 µg/ml denatured, fragmented salmon sperm DNA. Addition of 50% formamide lowers the prehybridization and hybridization temperatures to 42°C. Washes are performed in low salt concentration, e.g. in 2xSSC-0.5% SDS for 15 minutes at room temperature (RT, about 22°C), followed in 2xSSC-0.1% SDS at RT, and finally in 0.1xSSC-0.1% SDS at least at 65°C.

According to another preferred embodiment the NHSSP of the invention is encoded by an isolated nucleic acid molecule, which encodes a polypeptide comprising the amino acid sequence characterized in SEQ ID No. 2 or 4, or a polypeptide having at least 87% to the amino acid sequence SEQ ID No. 2 or 4. Preferred enzymes show at least 90%, preferably at least 95% identity. The identities of the two enzymes are compared within the corresponding sequence regions, e.g. within the mature or full-length region of the serine protease.

Thus, within the scope of the invention is the use of an isolated polypeptide having NHSSP activity, which is encoded by a nucleic acid molecule encoding the amino acid sequence of the full-length serine protease of the invention including the prepeptide (signal sequence) and the propeptide in addition to the mature form of the enzyme, and which amino acid sequence is characterized by SEQ ID No. 2 or 4, or a polypeptide having at least 87% identity to the amino acid sequence SEQ ID No. 2 or 4, respectively. Preferred enzymes show at least 90%, preferably at least 95% identity to the amino acid sequence SEQ ID No. 2 or 4, respectively.

The term "coding sequence" according to instant invention preferably means the nucleotide sequence which initiates from the translation start codon (ATG) and stops at the translation stop codon (TAA, TAG or TGA). The translated full-length polypeptide starts usually with methionine and comprises intron regions.

Also, within the scope of the invention is a NHSSP encoded by a nucleic acid molecule comprising the nucleotide sequence encoding the polypeptide SEQ ID No. 4, which encodes the mature NHSSP form, such as the polynucleotide of SEQ ID No. 3.

One embodiment of the invention is the NHSSP produced from an expression vector comprising the nucleic acid molecule, which encodes the NHSSP as defined above operably linked to regulatory sequences capable of directing the expression of said serine protease encoding gene in a suitable host, respectively, host cell.

Suitable hosts for the production of the NHSSP are homologous or heterologous hosts, such as microbial hosts including bacteria, yeasts and fungi.

The nucleic acid molecule of the invention may be RNA or DNA, wherein the DNA may constitute of the genomic DNA or cDNA.

Standard molecular biology methods can be used in isolation and enzyme treatments of the polynucleotide sequence encoding the neutral serine protease of the invention, including isolation of genomic and plasmid DNA, digestion of DNA to produce DNA fragments, sequencing, E. coli and P. pastoris transformations etc. The basic methods are described in the standard molecular biology handbooks, e.g. Sambrook and Russell, 2001 as well as in the user guide of Invitrogen™ named PichiaPink™ Expression System, Publication No MAN0000717 dated August 6, 2010 (Invitrogen, a company of ThermoFisher Scientific).

Further, within the scope of the present invention are nucleic acid molecules which encode a fragment of a NHSSP, wherein the fragment has serine protease activity and has at least 87% identity to the amino acid sequence SEQ ID No. 2 or 4. Preferred enzymes show at least 90%, preferably at least 95% identity to the amino acid sequence of SEQ ID No. 2 or 4. The identities of the two enzymes are compared within the corresponding sequence regions, e.g. within the full-length or mature region of the serine protease.

The nucleic acid molecule is preferably a molecule comprising the coding sequence as defined in SEQ ID No. 1, which encodes the full length form of the NHSSP of instant invention.

The isolated nucleic acid molecule of the invention may be a molecule comprising the coding sequence of the polynucleotide sequence contained in DSM 32492 as used in the cloning of the full length NHSSP gene.

The nucleic acid molecule of the invention may also be an analogue of the nucleotide sequence characterized in above. Analogues within the "degeneracy" of the genetic code preferably mean according to instant invention analogues of the nucleotide sequence, which differ in one or more nucleotides or codons, but which encode the functional protease of the invention.

Thus, within the scope of the invention is the use of an isolated nucleic acid molecule according to instant invention comprising a nucleotide sequence as defined in SEQ ID No. 1 or SEQ ID No. 3 and analogues thereof encoding the NHSSP of the invention.

The present invention relates also to a recombinant expression vector or recombinant expression construct, which can be used to propagate or express the nucleic acid sequence or gene encoding the NHSSP in a suitable prokaryotic or eukaryotic host. The recombinant expression vector comprises DNA or nucleic acid sequences which facilitate or direct expression and secretion of the NHSSP encoding sequence in a suitable host, such as promoters, enhancers, terminators (including transcription and translation termination signals) and signal sequences operably linked the polynucleotide sequence encoding said NHSSP. The expression vector may further comprise marker genes for the selection of the transformed strains or the selection marker may be introduced to the host in another vector construct by co-transformation. Said regulatory sequences may be homologous or heterologous to the production organism or they may originate from the organism, from which the gene encoding the NHSSP is isolated.

Examples of promoters for expressing the serine protease of the invention in fungal hosts are the promoters of A. oryzae TAKA amylase, alkaline protease ALP and triose phosphate isomerase, Rhizopus miehei lipase, Aspergillus nigeror A. awamori glucoamylase (glaA), Fusarium oxysporumtrypsin-like protease, Chrysosporium lucknowense cellobiohydrolase I promoter, Trichoderma reesei cellobiohydrolase I (Cel7A) etc.

In yeast, for example promoters of S. cerevisiae enolase (ENO-1), galactokinase (GAL1), alcohol dehydrogenase (ADH2) and 3-phosphoglycerate kinase can be used to provide expression. In Pichia pastoris using the Pichia Pink system expression is driven by the methanol inducible promotor of the alcohol oxidase (AOX).

Examples of promoter sequences for directing the transcription of the serine protease of the invention in a bacterial host are the promoter of lacoperon of Escherichia coli, the Streptomyces coelicoloragarase dagA promoter, the promoter of the B. licheniformis alpha-amylase gene (amyL), the promoter of the B. stearothermophilus maltogenic amylase gene (amyM), the promoters of the B. sublitis xylA and xylB genes, etc.

Suitable terminators include those of the above mentioned genes or any other characterized terminator sequences.

Suitable transformation or selection markers include those which complement a defect in the host, for example the dal genes from B. subtilisor, B. licheniformisor, Aspergillus amdS and niaD. The selection may be based also on a marker conferring antibiotic resistance, such as ampicillin, kanamycin, chloramphenicol, tetracycline, phleomycin or hygromycin resistance.

Extracellular secretion of the NHSSP of the invention is preferable. Thus, the recombinant vector preferably comprises sequences facilitating secretion in the selected host. The signal sequence of the NHSSP of the invention or the presequence or prepeptide may be included in the recombinant expression vector or the natural signal sequence may be replaced with another signal sequence capable of facilitating secretion in the selected host. Thus, the chosen signal sequence may be homologous or heterologous to the expression host.

Examples of suitable signal sequences are those of the fungal or yeast organisms, e.g. signal sequences from well expressed genes. Such signal sequences are well known by the person skilled in the art.

The recombinant vector may further comprise sequences facilitating integration of the vector into the host chromosomal DNA to obtain stable expression.

The present invention relates also to host cells comprising the recombinant expression vector as described above. Suitable hosts for production of the NHSSP are homologous or heterologous hosts, such as the microbial hosts including bacteria, yeasts and fungi. Production systems in plant or mammalian cells are also possible.

Also filamentous fungi such as Trichoderma, Aspergillus, Fusarium, Humicola, Chrysosporium, Neurospora, Rhizopus, Penicillium and Mortiriella are potential production hosts due to the ease of down-stream processing and recovery of the enzyme product. Suitable production systems developed for yeasts are systems developed for Saccharomyces, Schizosaccharomyces or Pichia pastoris. Suitable production systems developed for bacteria are a production system developed for Bacillus, for example for B. subtilis, B. licheniformis, B. amyloliquefaciens, for E. coli, or for the actinomycete Streptomyces.

The production host cell may be homologous or heterologous to the serine protease of the invention. The host may be free of homogenous proteases due to removal of proteases either by inactivation or removal of one or more host proteases, e.g. by deletion of the gene(s) encoding such homogenous or homologous proteases.

The present invention relates also to a process for producing an isolated polypeptide having NHSSP activity, said process comprising the steps of culturing the natural or recombinant host cell carrying the recombinant expression vector for a serine protease of the invention under suitable conditions and optionally isolating said enzyme. The production medium may be a medium suitable for growing the host organism and containing inducers for efficient expression. Suitable media are well-known to the person skilled in the art.

Instant invention relates to a polypeptide having NHSSP activity, said polypeptide being encoded by the nucleic acid molecule as defined above. Preferably, the polypeptide is a NHSSP obtained by culturing the host cell carrying the recombinant expression vector for a NHSSP of the invention.

The invention further relates to a process for obtaining an enzyme preparation comprising a polypeptide, which has NHSSP activity, said process comprising the steps of culturing a host cell carrying the expression vector of the invention and either recovering the polypeptide from the cells or separating the cells from the culture medium and obtaining the supernatant having NHSSP activity.

The present invention relates also to an enzyme preparation, which comprises the NHSSP of the invention. The enzyme preparation or composition has NHSSP activity and is obtainable by the process according to the invention. Within the invention is an enzyme preparation which comprises the NHSSP of the invention.

The enzyme preparation or the detergent preparation of the invention may further comprise different types of enzymes in addition to the NHSSP of this invention, for example another protease, amylase, lipase, cellulase, cutinase, pectinase, mannanase, xylanase and/or an oxidase such as laccase or peroxidase with or without a mediator. These enzymes are expected to enhance the performance of the NHSSP of the invention by removing components, which may be present in the material to be handled. Said enzymes may be natural or recombinant enzymes produced by the host strain or may be added to the culture supernatant after the production process.

The enzyme preparation of the invention may further comprise a suitable additive selected from the group of surfactants or surface active agent, buffers, anticorrosion agents, stabilizers, bleaching agents, mediators, builders, caustics, abrasives and preservatives, optical brighteners, antiredeposition agents, dyes, pigments, and Ca²⁺/Mg²⁺ chelating agents.

Surfactants are useful in emulsifying grease and wetting surfaces. The surfactant may be a non-ionic including semi-polar and/or anionic and/or cationic and/or zwitterionic.

Buffers may be added to the enzyme preparation to maintain or modify the pH or affect performance or stability of other ingredients.

Suitable stabilizers include polyols such as propylene glycol or glycerol, a sugar or sugar alcohol, lactic acid, boric acid, or boric acid derivatives, peptides, etc.

Bleaching agent is used to oxidize and degrade organic compounds. Examples of suitable chemical bleaching systems are H₂O₂ sources, such as perborate or percarbonate with or without peracid-forming bleach activators such as tetraacetylethylenediamine, or alternatively peroxyacids, e.g. of the amide, imide or sulfone type. Chemical oxidizers may be replaced partially or completely by using oxidizing enzymes, such as laccases or peroxidases.

Builders or complexing agents include substances, such as zeolite, diphosphate, triphosphate, carbonate, citrate, etc. The enzyme preparation may further comprise one or more polymers, such as carboxymethylcellulose, polyethylene glycol, polyvinyl alcohol, polyvinyl pyrrolidone, etc. Also, softeners, caustics, preservatives for preventing spoilage of other ingredients, abrasives and substances modifying the foaming and viscosity properties can be added.

In another embodiment of the invention said enzyme preparation is in the form of a liquid, powder or granulate, optionally in compressed form.

The NHSSP of the present invention may be used in the detergent, protein, brewing, bakery, meat, photographic, leather, dairy and pharmaceutical industries (Rao et al.,1998). It may further be used as an alternative to chemicals to convert fibrous protein waste to useful biomass, protein concentrate or amino acids (Anwar and Saleemuddin, 1998). The use of serine protease of the present invention may like other enzymes prove successful in improving leather quality and in reducing environmental waste and saving energy.

Subtilisin in combination with broad-spectrum antibiotics in the treatment of bums and wounds is known as an example of the use of serine proteases in pharmaceutical industry, therefore the NHSSP of the present invention may also find such use and may also like other proteases be applicable in the removal of blood on surgical equipments and cleaning contact lenses or dentures. Due to the effective pH optimum at around pH 6.8 it may also be used in a clinical environment to digest cell debris thus supporting wound healing. Blood has a pH between 7.3 and 7.4 thus very close to the one of NHSSP.

When wounds do not heal in an appropriate time the removal of fibrin cuffs and necrotic tissue do not allow infiltration of fibroblasts and therefore the onset of the wound healing process. It was found that the NHSSP according to the present invention has excellent properties in the preparation of wounds showing fibrin cuffs to promote their natural healing process by its biologically active proteolytic properties under physiological conditions. Therefore, it is another object of the invention to use the polypeptide having NHSSP activity according to the invention in the treatment of wounds, e.g., for debridement. Yet another object of the invention is the use of the polypeptide having NHSSP activity according to the invention in the manufacturing of a pharmaceutical composition for wound debridement, preferably together with at least one pharmaceutically acceptable carrier, excipient, diluent, cryoprotectant and/or lyoprotectant, e.g., for topical administration. Still another object of the invention is a pharmaceutical composition for wound debridement comprising the polypeptide having NHSSP activity according to the invention together with at least one pharmaceutically acceptable carrier, excipient, diluent, cryoprotectant and/or lyoprotectant, e.g., for topical administration.

Like alkaline protease from Conidiobolus coronatus, the NHSSP of the present invention may be used for replacing trypsin in animal cell cultures. The NHSSP of the invention can also be used in cleaning of membranes and destruction of biofilms. In baking the NHSSP of the invention can be used e.g. in destruction of the gluten network and in other food applications in hydrolysis of food proteins, e.g. milk proteins. The NHSSP of the invention can also be used, e.g. in the improvement of yeast, rendering (extracting more protein from animal bones), creating new flavors, reducing bitterness, changing emulsifying properties, generating bioactive peptides and reducing the allergenicity of proteins. The substrates of the NHSSP in this respect may include animal, plant and microbial proteins.

The present invention relates also to the use of the serine protease enzyme or the enzyme preparation for the manufacturing of detergent compositions for treating textile fibers, for treating wool, for treating hair, for treating leather or for the treatment of medical devices or implants for biofilm removal.

Another preferred embodiment of the invention is therefore the use of the NHSSP of the invention for the preparation, isolation or purification of nucleic acid molecules, e.g., in molecular biology. Here, the instant NHSSP shows excellent features in the nucleic acid purification process, wherein the NHSSP can be inactivated by a heat step to only 60 to 70 °C, thereby reducing time and energy efforts compared with the use of other proteases, e.g., Proteinase K (Example 5).

### EXAMPLES

### MATERIALS AND METHODS

All reagents and chemicals were obtained from Sigma-Aldrich, if not otherwise indicated. Photometric assays were performed using a Spectrophotometer NanoDrop™ ND-1000 (ThermoFisher Scientific Inc, NanoDrop Products, Wilmington, Delaware, USA). Protein concentration in solution was determined spectrophotometrically at 280nm. %-values mean % (w/v), if not otherwise indicated. UM means User Manual.

### Example 1: Cloning of the NHSSP derived from O. corvina, gene synthesis and preparation of the expression construct

Based on the amino acid sequence disclosed by Hyang et al. (2015) for protease 6877 of *Onygena corvina* (known from EMBL protein database KP290860) a protein with the native signal peptide and a C-terminal His6 tag was designed, thereby altering the genetic code for optimized expression in P. pastoris, and the de novo synthesis of the corresponding coding DNA strand (SEQ ID No. 1) was performed by GeneArt™ gene synthesis (ThermoFisher Scientific Inc., Waltham, Massachusetts, USA).

The obtained nucleotide sequence comprising SEQ ID No. 1 encoding the NHSSP derived from O. corvina of SEQ ID No. 2 was cloned into the Pichia pastoris expression vector pPink-HC (7667 kb, PichiaPink™ yeast expression system, Thermo Fisher Scientific, User Manual Publication Number MAN0000717 revision date 06.08.2010) into the restriction sites by endonucleases EcoRI (5'-end) and Kpnl (3'-end) using the primers according to the User Manual (UM) to result in expression vector pPink_HC-NHSSP.

Integrity of the expression cassette encoding NHSSP in pPink_HC-NHSSP was confirmed by DNA sequencing using forward primer P1 (5'-GACTGGTTCCAA TTGACAAGC) (SEQ ID No. 5) specific to AOX1 promoter and reversed primer P2 (5'-GCGTGAATGTAAGCGTGAC) (SEQ ID No. 6) specific to CYC1 terminator. The clone with the correct gene sequence was selected and plasmid DNA was amplified and purified using ExtractMe™ MidiPrep Purification Kit (DNA Gdansk/BLIRT SA, Gdansk, Poland) and further used for Pichia transformation according to the UM.

### Example 2A: Expression: Pichia transformation and clonal selection

Pichia transformation was performed with the gene construct obtained by Example 1 according to the method outlined in the UM of the PichiaPink™ yeast expression system, Thermo Fisher Scientific.

### 2.A.1: DNA preparation

To enable integration of the transgene into the P. pastoris genome, 10µg of the plasmid DNA obtained in Example 1 was linearized using Aflll restriction endonuclease within the TRP2 locus on pPink-HC vector.

### 2.A.2: Pichia Pink Competent Cell Preparation

The following PichiaPink™ strains from the Kit were used for transformation: Strain 1 (ade2), Strain 2 (ade2, pep4), Strain 3 (ade2, prb1), and Strain 4 (ade2, prb1, pep4). Competent Pichia cells were prepared according to the UM. Electroporation was performed with a Gene Pulser Xcell™ Electroporation System (Bio-Rad Laboratories, Inc., Hercules, California, USA) in 2mm gap cuvettes using Yeast pre-defined Program (V=2000V, C=25µF, R=200 Ohm, t=5ms). Before pulse, 80µl of cells were pre-incubated with 10µg of DNA of Example 2.1 on ice for 5 min. Immediately after electroporation 1 ml of ice-cold YPDS medium was added to the cells and then transferred to 30°C for 4h. After that, 100µl and 200µl of cells were plated on MGYA plates and grown for 3-4 days at 30°C. White colonies were selected for the screening of transgene expression.

### Example 2B: Monitoring of the Transgene Expression in Pichia

The expression level of the protease was analyzed in the culture medium of selected clones.

Cells were grown in 24-well plates. 1.5 ml of BMGY medium was inoculated with each clone of Pichia cells transformed with the expression vector pPink-HC and grown at 30°C in an orbital shaker at 250 rpm. After 24h the cells were centrifuged at 1500 g for 5 min. and re-suspended in 1 ml of BMMY medium containing 0.5% (v/v) methanol. After 48h culture media were analyzed for the presence of recombinant protein on Western blot by using His-tag-specific antibodies. After further 24h cultivation the cells were again supplemented with inductor amount to a final concentration of 0.5% (v/v) and the expression product again detected by Western blot. The recombinant NHSSP obtained with PichiaPink™ Strain 1 (ade2) exhibited an apparent molecular weight of 34 kDa in the SDS-PAGE.

Example 2C: Up-scaling of the Expression in Pichia of PichiaPink™ Strain 1 (ade2) Scaling-up of the protein expression was performed in a Sartorius Biostat C bioreactor in 6 I to 8 I batches in Basal Salt Medium (BSM according to the UM) and obtained a yield of more than 300 mg of the mature NHSSP product. Fermentation conditions were used as outlined in the PichiaPink™ Expression UM (cf. above) using 0,5% (v/v) methanol as AOX1 promoter inductor as described. Success of expression was again monitored as described in Example 2B. Peak expression was observed about 80h post induction.

### EXAMPLE 3. Purification of the Onygena corvina protease

The expression product of Example 2C was isolated and further purified by ammonium sulfate precipitation, ultrafiltration and hydrophobic interaction chromatograpy.

### Example 3A: Ammoniumsulfate precipitation

BSM medium with NHSSP was filtered 0.2µm and subjected for stepwise ammonium sulfate precipitation. Solid ammonium sulfate reagent was added to a final concentration of 40% saturation, stirred at 4°C and after 1 hour precipitated material was removed by centrifugation at 18,000 g for 30 min. and discarded. To the remaining supernatant solid ammonium sulfate was further added to a final concentration of 80% and after 1 h the precipitate was recovered by centrifugation at 18,000 g for 30 min. The pellet containing active NHSSP was resuspended in Resuspension buffer (50mM buffer NaH₂PO₄/Na₂HPO₄, 50mM NaCl, pH 6.6) and dialyzed overnight against Resuspension buffer in excess of 1:250 (v/v).

### Example 3B: Hydrophobic Chromatography

A purification strategy was developed using Phenyl-Sepharose 6FastFlow (low sub) resin (GE Healthcare, Cat. No 17-0965-99).

5 liters of NHSSP post expression BSM medium was filtered 0.22 µm. The protein containing medium was concentrated to 500 ml using Vivaflow 10 kDa MWCO PES filtered cassette (Sartorius) and 30x times buffer was exchanged to the final Buffer A (25mM Na₂HPO₄/NaH₂PO₄, pH 6.6, 50mM NaCI).

At the end of buffer exchange, solid (NH₄)₂SO₄ was added to final concentration of 2 M and the solution was filter-sterilized of a pore size of 0.22 µm. NHSSP solution was loaded into Phenyl-Sepharose resin (100 ml packed in XK50 column). Loading flow rate was 10 ml/min. Unbound protein was washed from the column with 1 I (10CV) Buffer A (25mM NaH₂PO₄/Na₂HPO₄ pH 6.6, 50 mM NaCl, 2M (NH₄)₂SO₄). NHSSP was eluted with 400 ml (4CV) of Buffer B (25 mM NaH₂PO₄/Na₂HPO₄ pH 6.6, 50 mM NaCI) using elution profile: 30%, 50%, 70%, 85%, and 100% B. Fractions of 20 ml were collected and analyzed on SDS-PAGE and Western blot for content of NHSSP protein. Fractions containing NHSSP protein were pooled. Purified NHSSP was stored at a concentration of 0.4 to 1 mg/ml in NaH₂PO₄/Na₂HPO₄, 100mM sodium chloride, pH 6.6, glycerol 10% (for storage at -80°C) or glycerol 50% (for storage at -20°C) before further use.

### EXAMPLE 4. Characterization of the Onygena corvina protease

The NHSSP obtained according to Example 3B was subsequently characterized.

### Example 4A. Determination of the molecular weight by SDS-PAGE

The purified NHSSP was analyzed on 12% SDS-PAGE and identified by Western blot using nitrocellulose membrane and anti-His6-antibodies and the gel was stained with Coomassie blue. The results indicated an apparent molecular mass of NHSSP of about 34 KDa.

### Example 4B. Determination of the molecular weight by N- and C-terminal sequencing and peptide mass fingerprinting (PMF) for determination of the protein identity and the C-terminal fragment

Aliquots containing 4 µg of the protein sample for the tryptic digest and for AspN digest were reduced with dithiothreitol, alkylated with iodoacetamide and digested with either sequencing grade trypsin (Serva) or endoproteinase Asp-N (Roche Diagnostics) at 37°C overnight. The peptides were analyzed by MALDI-TOF-MS operating in reflectron mode. As matrix alpha-cyano-4-hydroxycinnamic acid was used. The measured peptide masses were compared with the provided sequences using the software Mascot (Matrix Science). Identification threshold was set to p<0.05; peptide mass tolerance was set to 100 ppm. Carbamidomethylated cysteines were set as fixed modifications; oxidized methionines were set as variable modifications. Additionally, Mascot database searches using the SwissProt database with sequences of all species were performed. The C-terminus according to this method is suggested to be Leu-Leu-Tyr-Asn-Gly-Ser.

For N-terminal sequencing 6 µg of the sample were diluted in 100 µl 0.1% trifluoroacetic acid and immediately adsorbed on a ProSorb PVDF cartridge (Applied Biosystems), washed with 100 µl HPLC-grade water, air dried and transferred to the sequencer cartridge. Sequencing was performed on a Procise 494 Sequencer (Applied Biosystems). The standard method "Pulsed-Liquid" was used. The sample was sequenced for 8 cycles of Edman degradation. At the beginning of the sequencing run, a blank gradient and a standard mixture containing 10 pmol of the PTH-derivatives of each of the 19 proteinogenic amino acids (without cysteine) was analyzed. The amino acids from each sequencing cycle were identified by their retention time and quantitated by comparison of peak heights with the standard chromatogram. The N-Terminus of the mature NHSSP was determined to be Ala-Leu-Thr-Thr-Gln-Pro-Asn-Ala.
Taking the N- and the C-terminal amino acids into account as the borders of the mature enzyme a molecular weight of 28.4 kDa was calculated. This value is significantly lower than the molecular weight of 34 kDa as determined by SDS-PAGE analysis. However, the NHSSP did not react to anti-His6-antibodies supporting the finding by mass fingerprint that the His6-tag is abandoned during maturation of the enzyme.

### Example 4C. NHSSP Assay Performance

NHSSP was essentially analyzed for its proteolytic activity as described by Huang Y et.al. using azocasein as substrate. The reaction mixture contained 100 µl diluted enzyme solution and 100 µl 0.5% azocasein dissolved in 50 mM potassium phosphate buffer (pH 6.5). The reaction was carried out at 42°C for 50 min with constant agitation at 300 rpm using a TS-100 Thermo-Shaker, SC-20 (Biosan Ltd). After incubation, the reaction was stopped by adding 300 µl of 0.6 M trichloroacetic acid (TCA) and centrifuged at 10000 g for 10 min to remove the substrate. 100 µl supernatant was transferred to a microtiter plate and absorbance was measured at 450 nm using a plate reader. As a control, 100 µl 0.5% azocasein dissolved in the same buffer (of the sample) was added to 300 µl of 0.6M TCA before the addition of 100 µl of enzyme solution. The control mixture was incubated at 42°C for 50 min and treated in the same way as the sample.

### Example 4.C.1. Determination of the pH-optimum

For the determination of the isolated NHSSP activity at different pH-values, the enzymatic hydrolysis of azocasein was measured in Britton-Robinson buffer (Britton et al., 1931) at 30°C for 30 minutes. The reaction was stopped by the addition of TCA to a final concentration of 293mM, the mixture was centrifuged as described above and the absorbance of the supernatant was measured at 450nm. NHSSP exhibits the highest enzymatic activity at pH 6.5 as shown in Table 1.

**Table 1: Effect of the pH on the enzymatic activity of NHSSP.**

| **pH-Value** | **OD₄₅₀** | **Activity (%)** |
|---|---|---|
| **3.0** | **0.156** | **0,9** |
| **3.5** | **0.148** | **-0,9** |
| **4.0** | **0.201** | **10,7** |
| **4.5** | **0.378** | **49,1** |
| **5.0** | **0.566** | **90,0** |
| **5.5** | **0.610** | **99,6** |
| **5.5** | **0.614** | **100** |
| **6.5** | **0.561** | **88,9** |
| **7.0** | **0.439** | **62,4** |
| **7.5** | **0.381** | **49,8** |
| **8.0** | **0.347** | **42,4** |
| **8.5** | **0.287** | **29,4** |
| **9.0** | **0.220** | **14,8** |
| **9.5** | **0.208** | **12,2** |
| **10.0** | **0.156** | **0,9** |

### Example 4.C.2. Determination of the temperature-optimum

Temperature-dependent proteolytic activity of NHSSP was determined by using Phenyl-Sepharose Chromatography purified enzyme. The NHSSP was assayed as described above using azocasein at a constant pH 6.5 in phosphate buffer and different temperatures from 10 to 70°C. The results are shown in Table 2.

**Table 2: Effect of Temperature on enzymatic activity of NHSSP.**

| **Temperature (°C)** | **OD₄₅₀** | **Activity (%)** |
|---|---|---|
| **10** | **0.015** | **7.0** |
| **15** | **0.017** | **8.9** |
| **20** | **0.027** | **18.3** |
| **25** | **0.037** | **27.7** |
| **30** | **0.067** | **55.9** |
| **35** | **0.092** | **79.3** |
| **40** | **0.114** | **100.0** |
| **45** | **0.112** | **98.1** |
| **50** | **0.049** | **39.0** |
| **55** | **0.023** | **14.6** |
| **60** | **0.010** | **2.3** |
| **65** | **0.007** | **-0.5** |
| **70** | **0.008** | **0.5** |

The temperature optimum for NHSSP was determined at 40°C, a fast decrease in activity was observed above 55°C.

### Example 4.C.3. Heat inactivation of NHSSP

Temperature-dependent inactivation of NHSSP was determined under conditions outlined in 4.C.2 at two different temperatures. Results are shown in Table 3.

**Table 3: Heat inactivation of NHSSP at 50°C and 60°C**

| **Time (minutes)** | **OD₄₅₀** | **Activity (%)** | **OD₄₅₀** | **Activity (%)** |
|---|---|---|---|---|
| | **Temperature 50°C** | | **Temperature 60°C** | |
| **0** | **0.218** | **100.0** | **0.197** | **100.0** |
| **1** | **0.207** | **94.9** | **0.167** | **84.9** |
| **5** | **0.176** | **80.6** | **0.038** | **0.0** |
| **10** | **0.088** | **39.8** | **0.000** | **0.0** |
| **15** | **0.016** | **6.5** | **0.000** | **0.0** |
| **20** | **0.007** | **2.3** | **0.000** | **0.0** |
| **25** | **0.002** | **0.0** | **0.000** | **0.0** |
| **30** | **0.002** | **0.0** | **0.000** | **0.0** |
| **40** | **0.002** | **0.0** | **0.000** | **0.0** |

### Example 4.C.4. Inhibitors of NHSSP

The influence of different inhibitors on the enzymatic activity of NHSSP was determined by hydrolysis of azocasein in Britton-Robinson buffer pH 6.5 supplemented with appropriate substances. The reaction was stopped by the addition of TCA to the final concentration of 293 mM, centrifuged as described above, and the absorbance of supernatants was measured at 450 nm.

The enzymatic reaction was carried out at 40°C with a final inhibitor concentration of 1 mM. PMFS was identified as an inhibitor of NHSSP, whereas EDTA, TCEP, and ß-mercaptoethanol did not significantly inhibit protease activity.

**Table 4: Effect of protease inhibitors on the enzymatic activity of NHSSP**

| **Inhibitor** | **Activity (%)** |
|---|---|
| **None** | **100** |
| **TCEP** | **97** |
| **EDTA** | **100** |
| **PMSF** | **40** |
| **2-ß ME** | **99** |

### Example 4.C.5. Specific activity of NHSSP and Proteinase K (from Tritirachium album)

Purified NHSSP was assayed according to the assay protocol described in Example 4.C.2 with the exception that a constant temperature of 42°C was used. The protein amount was determined according to the absorbance at 280nm.

The specific activity was calculated according to the manufacturer's information (Merck-Millipore, Darmstadt, Germany) using the formula: Protease (Units/mg) = (140 x Abs. (440 nm) - 4) x 1mg/concentration of enzyme in the assay/1000.

Linear absorbance range was 0.1 to 1.0, with r = 0.99. The results of 3 independent assays show the specific activities of NHSSP and Proteinase K and are given in Table 5.

**Table 5: Specific activity of NHSSP**

| **Protease Specific Activity* (Units/mg)** | | |
|---|---|---|
| **Protease** | **Average** | **Standard Deviation** |
| **NHSSP/HIC** | **65,8** | **18,2** |
| | **61,3** | **15.5** |
| | **64,7** | **18,0** |
| **Proteinase K** | **32,3** | **5.5** |
| | **31,7** | **5.5** |
| | **31,6** | **5.0** |

The results show that NHSSP has about the 2-fold specific activity of Proteinase K.

### EXAMPLE 5. Applications of the NHSSP

### Example 5A. Protection of DNA from DNAse digestion

As positive control, each chromosomal and plasmid DNA was fully digested by DNAse at a concentration of 50 µg/ml DNAse for 30 min at 37°C in a suitable buffer. NHSSP was added to DNA and subsequently the amount of DNAse was added to reach a ratio of protease/DNAse of from 1:1 to 5:1 and the samples were incubated for 1, 10 or 30 minutes at 37°C in the presence and absence of 0,5% SDS.

The results indicate that the heat-sensitive neutral serine protease can effectively protect chromosomal and plasmid DNA from DNAse digestion. This protective effect was fully maintained after the protease solution was stored for 24 h at 4°C.

Accordingly, it is indicated that the heat-sensitive neutral serine protease provides specific advantages compared with the use of proteinase K in the preparation and isolation of DNA, e.g, in molecular biology. Main advantages are due to the higher specific activity of the neutral serine protease and its possible deactivation at relatively low temperatures of 50-65°C thus for example avoiding melting of primers from DNA in a PCR amplification reaction. Finally, in many cases even a deproteination step can be avoided when using the heat-sensitive neutral serine protease of O. corvina in the purification of nucleic acid molecules.

Example 5B. Processing of wheat flour for the manufacturing of food products A dough comprising wheat flour is prepared thereby adding NHSSP in an amount of 0.02 to 0.5 % (w/w). The dough is processed at ambient temperature of 25 to 40°C for further 15 minutes to 12 hours.

The results indicate that NHSSP considerably reduces the amount of gluten in dough, thereby providing advantages in the preparation of gluten-free or gluten reduced bakery products.

### Example 5C. Detergent compositions

A detergent composition is prepared thereby adding NHSSP in an amount of 0.01 to 0.5 % (w/w). The composition is used in the treatment of stained fibers at ambient temperature of 25 to 40°C for 30 minutes to 2 hours.

The results indicate that NHSSP provides excellent results in the removal of proteinaceous stains from textile fibers after application in detergent compositions.

### REFERENCES

Anwar, A and M Saleemuddin, Bioresource Technology, 1998, 64:175-183.
Britton, H.T.S. and Robinson, R.A., J. Chem. Soc., 1931, 1456-1462
Bolton and McCarthy, PNAS USA, 1962, 48, 1390
Chen, Y-J, and M Inouye, Curr. Opin. Struct. Biol., 2008, 18:765-770.
Edman P and G Begg, Eur. J. Biochem., 1967, 1:80-91.
Gasteiger et al., Nucleic Acids Res., 2003, Jul 1;31(13):3784-8
Gupta, R, et al. Appl. Microbiol. Biotechnol., 2002, 60: 381-395.
Hyuang Y. et al., Appl. Microbiol. Biotechnol., 2015, 99: 9635-9649.
Laemmli, UK, Nature, 1970, 227:680-685.
Rao, MB et al., Mol. Biol. Rev., 1998, 62:597-635.
Sambrook J and Russell DW, 2001. Molecular cloning, a laboratory manual. Cold Spring Harbor Laboratory, New York, USA.
Shimogaki, H et al. Agric. Biol. Chem., 1991, 55:2251-2258.

## Claims

1. A process for the preparation of an isolated polypeptide having a calculated molecular weight of 25.6 to 31.2 kDa and neutral heat-sensitive serine protease (NHSSP) activity, said process comprising the steps of
(a) culturing a host cell comprising a nucleic acid molecule, which comprises a polynucleotide sequence encoding a NHSSP selected from the group consisting of:
(i) a nucleic acid sequence encoding a polypeptide comprising the amino acid sequence of SEQ ID No. 2 or 4;
(ii) a nucleic acid sequence encoding a polypeptide having at least 87% identity to the amino acid sequence of SEQ ID No. 2 or 4;
(iii) a nucleic acid sequence comprising the coding sequence of the nucleotide sequence of SEQ ID No. 1 or 3;
(iv) a nucleic acid sequence comprising a coding sequence which differs from the coding sequence of the nucleic acid sequence of (iii) due to the degeneracy of the genetic code; and
(v) a nucleic acid sequence encoding a polypeptide having serine protease activity as deposed under DSM Accession No. 32492;
(vi) a nucleic acid sequence which hybridizes under stringent conditions to the nucleic acid sequence as defined in (iii) to (v); and
(b) recovering said polypeptide having serine protease activity from the culturing broth;
(c) maturing said polypeptide or said recovered polypeptide; and
(d) isolating said matured polypeptide having NHSSP activity.

2. The process according to claim 1, wherein an expression vector comprises said polynucleotide sequence, which is operably linked to regulatory sequences capable of directing expression of said polypeptide in a host cell and, optionally, wherein said polynucleotide sequence furthermore encodes a signal peptide or tag peptide which facilitates the purification of said polypeptide in said recovery step (b) or isolation step (d).

3. The process according to claim 1 or 2, wherein said host cell is eukaryotic, preferably derived from fungus or yeast.

4. An isolated polypeptide having a calculated molecular mass of 25.6 to 31.2 kDa and exhibiting a neutral heat-sensitive serine protease (NHSSP) activity obtainable by the process according to any of claims 1 to 3, **characterized in that** the protease activity of said polypeptide has a pH optimum of pH 5.0 to 7.5 at 30°C in 20mM potassium phosphate buffer using 30 min reaction time and azocasein as a substrate.

5. The polypeptide according to claim 4, **characterized in that** the protease activity of said polypeptide has a temperature optimum of 35 to 45°C at pH 5.5 in 50mM potassium phosphate buffer using 30 min reaction time and azocasein as a substrate.

6. The polypeptide according to claim 4 or 5, which is SEQ ID No. 2 or a polypeptide having at least 87% sequence identity with the polypeptide defined by SEQ ID No. 2.

7. An enzyme preparation comprising the polypeptide having NHSSP activity according to any of claims 4 to 6, optionally comprising an effective amount of a suitable additive selected from the group of stabilizers, buffers, surfactants, and preservatives.

8. A detergent preparation comprising the polypeptide having NHSSP activity according to any of claims 4 to 6 or the enzyme preparation according to claim 7, optionally comprising an effective amount of a suitable additive selected from the group of stabilizers, buffers, enzymes, and preservatives.

9. The preparation according to any of claims 7 to 8, which is in the form of liquid, powder or granulate, optionally compressed.

10. The use of an isolated polypeptide having NHSSP activity selected from
(i) the amino acid sequence as defined in SEQ ID No. 2 or 4;
(ii) the amino acid sequence having at least 87% identity to the amino acid sequence of SEQ ID No. 2 or 4;
(iii) the amino acid sequence encoded by the polynucleotide as defined in SEQ ID No. 1 or 3;
(iv) the amino acid sequence encoded by a polynucleotide which differs from the coding sequence of SEQ ID No. 1 or 3 due to the degeneracy of the genetic code;
(v) the amino acid sequence encoded by the polynucleotide encoding a polypeptide having serine protease activity as deposited under DSM Accession No. 32492;
(vi) the amino acid sequence encoded by a polynucleotide encoding a polypeptide having serine protease activity which hybridizes under stringent conditions to the nucleic acid molecule as defined in (iii) to (v);
in a proteolytic process at a pH value of between pH 5.0 and pH 7.5.

11. The use of the isolated polypeptide having NHSSP activity according to claim 10 at a proteolytic process temperature of between 20 and 45°C.

12. The use of a polypeptide having NHSSP activity according to any of claims 4 to 6 or the preparation according to claims 7 for the processing or preparation of food or feed products.

13. The use of a polypeptide having NHSSP activity according to any of claims 4 to 6 or the preparation according to any of claims 7 to 9 in the manufacturing of a cleaning or washing product for the degradation of proteinaceous material.

14. The use of a polypeptide having NHSSP activity according to any of claims 4 to 6 or the preparation according to any of claims 7 to 9 in the purification of nucleic acid molecules.

15. The use of a polypeptide having NHSSP activity according to any of claims 4 to 6 or the preparation according to any of claims 7 to 9 in the manufacturing of a pharmaceutical composition for wound debridement, optionally together with at least one pharmaceutically acceptable carrier, excipient, diluent, cryoprotectant and/or lyoprotectant, optionally, for topical administration.

16. A method of performing a proteolytic process at a pH value of pH 5.0 to 7.5 and at a temperature of 20 to 50°C, wherein a polypeptide of a calculated molecular mass of 25.6 to 31.2 kDa having NHSSP activity is employed, optionally in the presence of one or more detergents, comprising the polypeptide selected from the group consisting of
(i) the amino acid sequence as defined in SEQ ID No.4; and
(ii) the amino acid sequence having at least 87% identity to the amino acid sequence of SEQ ID No.4;
said method optionally further comprising the step of terminating said proteolytic process by deactivating said protease at a temperature of 55°C or more.
